# EUROPÄISCHE PATENTANMELDUNG

(11) **EP 0 552 773 A1**
(43) Veröffentlichungstag der Anmeldung: **28.07.1993**
(21) Anmeldenummer: 93100907.0
(22) Anmeldetag: 21.01.1993
(51) Int. Cl.: C09D 5/00, C07C 63/70, C07C 63/72

(54) **Beschichtung von Substratoberflächen**

(30) Priorität: 23.01.1992 DE 4201800
(71) Anmelder: WACKER-CHEMIE GMBH, D-81737 München (DE)
(72) Erfinder: Roth, Michael, Dr., W-8263 Burghausen (DE); von Au, Günther, Dr., W-8263 Burghausen (DE); Blümlhuber, Chista, W-8261 Neuötting (DE); Loher, Karl-Heinz, W-8263 Burghausen (DE)

(57) **Zusammenfassung**

In dem Verfahren zur Beschichtung von nicht wassersaugenden Substratoberflächen werden die Substratoberflächen mit einer durch Behandlung mit Wasser wieder entfernbaren wässrigen Zusammensetzung beschichtet, die eine wäßrige Lösung, Dispersion und/oder Emulsion filmbildender Stoffe sowie wasserabweisender Stoffe umfaßt. Das Verfahren eignet sich auch zur Beschichtung von porösen, wassersaugenden Substraten, wenn diese vor der Beschichtung mit der wässrigen Zusammensetzung wasserabweisend behandelt werden.

## Beschreibung

Die Erfindung betrifft ein Verfahren zur Beschichtung von Substratoberflächen mit einer wässrigen Zusammensetzung filmbildender Stoffe sowie wasserabweisender Stoffe. Die Beschichtung dient zum Schutz und zur leichten Reinigung der Oberflächen.

Die Entfernung von Verunreinigungen, wie Farbe und Taubenschmutz auf Substraten wie Metallen, Kunststoff, lackierten Flächen, Holz, Beton und Stein, ist bei der Anwendung mechanischer Verfahren, wie Bestrahlen mit Feststoffpartikeln, mit einer Beschädigung der Substratoberflächen verbunden.

Der Einsatz von Säure, Basen oder organische Lösungsmittel enthaltenden Reinigungsmitteln ist in sehr vielen Fällen wegen dem dadurch verursachten Angriff der zu reinigenden Substratoberfläche nicht möglich.

Die Reinigung mit Kalt- oder Heißwasser oder Wasserdampf, gegebenenfalls unter Anwendung von Druck, führt, insbesondere bei offenporigen Substraten, wie Baustoffen, zu einer starken Wasserbelastung des Substrats mit Folgeschäden.

Verschiedene bekannte Verfahren ermöglichen eine leichtere Reinigung durch eine Präventivbehandlung der Substratoberflächen.

Aus der Praxis ist bekannt, die Substratoberflächen mit einer konzentrierten Lösung organischer Polymere zu behandeln. Diese als Versiegelung bezeichneten Behandlungen sind nicht nur ungenügend wirksam, sondern verstopfen im Falle poröser Substrate die Poren, so daß die Luft- und Wasserdampfdurchlässigkeit behindert wird. Die Versiegelungen führen oft auch zu ungünstigen optischen Veränderungen der Substratoberflächen, wie Glanz oder Dunkelfärbung. Die in der Praxis verwendeten Lösungen organischer Polymere enthalten überwiegend organische Lösungsmittel, die gesundheitsschädlich, feuergefährlich und umweltbelastend sind. Die Entfernung von Verunreinigungen von so vorbehandelten Substratoberflächen kann meistens nur mit organischen Lösungsmitteln erfolgen.

Eine die Luft- und Wasserdampfdurchlässigkeit weniger behindernde öl- und wasserabweisende Imprägnierung ist in DE-A-25 26 287 (M. Roth und H. Glück; offengelegt für Wacker-Chemie GmbH am 30.12.1976) beschrieben. Die Substratoberflächen werden bei diesem Verfahren mit einer Kombination aus Organosiliciumverbindungen und fluorhaltigen organischen Verbindungen behandelt. Die Entfernung von Verunreinigungen erfordert jedoch die Verwendung von organischen Lösungsmitteln.

In DE-C-36 30 520 (H. Ramesohl; ausgegeben am 17.12.1987) ist ein Verfahren zur Imprägnierung von Stein bekannt, bei dem auf den imprägnierten Stein eine farbaufnehmende ablösbare Beschichtung, die die Imprägnierung nicht durchdringen kann, aufgetragen wird. Im Ausführungsbeispiel wird die Steinoberfläche öl- und wasserabweisend imprägniert und darauf eine Beschichtung aus durch Lösungsmittel verflüssigten Hartwachsen aufgetragen. Die Wachsbeschichtung beeinträchtigt jedoch die Luft- und Wasserdampfdurchlässigkeit so sehr, daß das Austrocknungsverhalten des mit diesem Verfahren behandelten Steins verschlechtert wird, was zu Schäden führen kann. Ferner muß das abgelöste Wachs als umweltbelastender Stoff als Sondermüll entsorgt werden.

Weiterhin ist aus der Praxis bekannt, wäßrige Lösungen von Polysacchariden auf die Substratoberfläche aufzutragen und diese Schicht bei Bedarf zusammen mit den Verunreinigungen mit Wasser abzuspülen. Dieses Verfahren hat sich insbesondere wegen der leichten Wasserlöslichkeit der Polysaccharidschicht auf beregneten Außenflächen nicht bewährt. Die Polysaccharidschicht ist weder wasser- noch ölabweisend und schützt das Substrat nicht gegen die Aufnahme von Wasser oder organischen Flüssigkeiten, wie Fette und Öle.

Aufgabe der vorliegenden Erfindung ist es ein Verfahren zur Beschichtung von nicht wassersaugenden Substratoberflächen bereitzustellen, wobei die Beschichtung luft- und wasserdampfdurchlässig und ausreichend witterungsbeständig sein soll und zusammen mit den Verschmutzungen ohne organische Lösungsmittel leicht und rückstandsfrei entfernbar sein soll und weder Glanz noch Dunkelfärbung aufweisen soll, wobei das Verfahren auch auf einem wasserabweisend imprägnierten porösen Substrat anwendbar sein soll.

Die Erfindung betrifft ein Verfahren zur Beschichtung von nicht wassersaugenden Substratoberflächen, wobei die Substratoberflächen mit einer durch Behandlung mit Wasser wieder entfernbaren wässrigen Zusammensetzung beschichtet werden, die eine wäßrige Lösung, Dispersion und/oder Emulsion filmbildender Stoffe sowie wasserabweisender Stoffe umfaßt.

Die filmbildenden Stoffe und die wasserabweisenden Stoffe sind gegenüber den zu beschichtenden Substraten inert. Damit die Beschichtung wieder mit Wasser entfernt werden kann, dürfen die filmbildenden Stoffe und die wasserabweisenden Stoffe weder mit sich selbst, noch mit einem anderen Bestandteil der Beschichtung vernetzen oder sich mit dem Substrat umsetzen.

Die wässrigen Zusammensetzungen enthalten vorzugsweise keine mit Wasser nicht mischbaren organischen Lösungsmittel. Sie enthalten maximal 10 Gew.-%, vorzugsweise 0 bis 5 Gew.-%, mit Wasser mischbare Lösungsmittel oder Lösungsmittelgemische, damit lackierte oder imprägnierte Substratoberflächen nicht angegriffen werden. Physiologisch unbedenkliche Lösungsmittel sind bevorzugt. Falls Lösungsmittel verwendet werden, sind Lösungsmittel oder Lösungsmittelgemische mit einem Siedepunkt bzw. Siedebereich von bis zu 160°C bei 0,1 MPa bevorzugt. Beispiele für solche Lösungsmittel sind Alkohole, wie Methanol, Ethanol, n-Propanol, iso-Propanol; Ether, wie Dioxan; Glykolether, wie Diethylenglycoldimethylether, Ethylenglykolmonomethylether, Ethylenglykolmonoethylether; Ketone, wie Aceton; ether-gruppenhaltige Ester, wie Essigsäure-(ethylenglykolmonomethylether)ester.

Bevorzugte filmbildende Stoffe sind organische synthetische Polymere wie Polyvinylchlorid, Polyethylen, Polypropylen, Polyvinylacetat, Polyvinylalkohol, Polycarbonat, Polyacrylat, Polymethacrylat, Polymethylmethacrylat, Polystyrol, Polyacrylnitril, Polyvinylidenchlorid, Polyvinylfluorid, Polyvinylidenfluorid, Polyvinylidencyanid, Polybutadien, Polyisopren, Polyether, Polyester, Polyamid, Polyurethan, Polyimid, Silikone, Polyvinylpyrrolidon, Polyacrylamid, Polyethylenglykol und deren Derivate und ähnliche Polymere einschließlich Copolymere sowie natürliche Polymere wie Cellulose, Stärke, Casein und natürliches Gummi, sowie halbsynthetische hochmolekulare Verbindungen wie Cellulosederivate, z. B. Methylcellulose, Hydroxymethylcellulose und Carboxymethylcellulose und deren Natriumsalze.

Besonders bevorzugte filmbildende Stoffe sind wasserlöslich oder mit Wasser quellbar, wie Polysaccharide und Polyvinylalkohol.

Als Polysaccharide können beispielsweise Cellulose, die vorstehend genannten Cellulosederivate, Celluloseether und cyclische Polysaccharide, wie Cyclodextrine, verwendet werden.

Der Polyvinylalkohol hat vorzugsweise Verseifungszahlen von 800 bis 0 entsprechend dem Verbrauch von mg KOH/1g Polyvinylalkohol (die Verseifungszahl 0 entspricht 100% OH-Gruppen). Besonders bevorzugt sind Verseifungszahlen von 450 bis 0, insbesondere von 200 bis 0. Die Viskosität der 4%-igen wässrigen Lösung eines bevorzugten Vinylalkohols beträgt bei 20°C 1 bis 200 mPa.s, insbesondere 3 bis 60 mPa.s.

Der Polyvinylalkohol kann neben Vinylacetateinheiten auch noch 0 bis zu 40 Mol-% gleicher oder verschiedener Einheiten aus der Gruppe der Acrylsäureester, Methacrylsäureester, Vinylester, Olefine, Vinylaromaten, ethylenisch ungesättigter Carbonsäuren und deren Amide enthalten. Bevorzugt ist jedoch ein Gehalt von 0 bis 10 Mol-%, insbesondere 0 bis 1 Mol-%, bezogen auf das Gesamtgewicht des Polymerisats. Die Estergruppen der vorstehenden Einheiten sind vorzugsweise mindestens teilweise zu den entsprechenden Säuresalzgruppen und Vinylalkoholgruppen hydrolysiert.

Bevorzugte (Meth)acrylsäureester, die in Copolymeren mit Vinylacetat gegebenenfalls hydrolysiert werden oder auch als Homopolymere eingesetzt werden können, sind die Ester von Alkoholen mit 1 bis 10 Kohlenstoffatomen, wie Methyl(meth)acrylat, Ethyl(meth)acrylat, Propyl(meth)acrylat, Butyl(meth)acrylat, 2-Ethylhexyl(meth)acrylat oder Lauryl(meth)acrylat.

Bevorzugt zu nennende Vinylester sind die Ester von Alkylcarbonsäuren mit 1 bis 15 C-Atomen, wie Vinylacetat, Vinylpropionat, Vinylbutyrat, Vinyl-2-ethylhexanoat, Vinyllaurat oder 1-Methylvinylacetat (Isopropenylacetat).

Geeignete Olefine sind Ethylen, Propylen oder Butadien. Bevorzugte Vinylaromaten sind Styrol oder Vinyltoluol.

Beispiele für ethylenisch ungesättigte Carbonsäuren und deren Amide sind Acrylsäure, Methacrylsäure, Itakonsäure, Fumarsäure, Maleinsäure sowie deren Monoamide und Diamide, insbesonders (Meth)acrylsäure, (Meth)acrylsäureamid und N-Methylolacrylamid.

Beispiele für Copolymerisate, die gegebenenfalls vollständig oder teilweise hydrolysiert als filmbildende Stoffe eingesetzt werden können, sind Vinylacetat-Ethylen-Copolymerisate, Vinylacetat-Ethylen-N-Methylol(meth)acrylamid-Terpolymerisate, Alkylacrylat-N-Methylol(meth)acrylamid-Copolymerisate und Vinylacetat-Ethylen-Acrylat-Mischpolymerisate oder Vinylacetat-Acrylat-Mischpolymerisate, die gegebenenfalls einen N-Methylol(meth)acrylamid-Anteil aufweisen.

Vorzugsweise werden filmbildende Stoffe eingesetzt, von denen wässrige Lösungen herstellbar sind.

Die wässrige Zusammensetzung umfaßt vorzugsweise 0,1 bis 20 Gew.-%, insbesondere 1,0 bis 10 Gew.-%, filmbildende Stoffe.

Im erfindungsgemäßen Verfahren können beliebige wasserabweisende Stoffe eingesetzt werden. Wenn die wasserabweisenden Stoffe nicht wasserlöslich und nicht emulgierbar sind, müssen sie so feinteilig eingesetzt werden, daß sie dispergierbar sind. Die feinteiligen wasser

abweisenden Stoffe haben vorzugsweise eine Oberfläche von mindestens 20 m²/g.

Im erfindungsgemäßen Verfahren können als wasserabweisende Stoffe beispielsweise hydrophobierte anorganische Stoffe oder gegebenenfalls fluorierte Wachse, Paraffine, Carbonsäuresalze, organische oder siliziumorganische polymere Verbindungen eingesetzt werden.

Beispiele geeigneter hydrophobierter anorganischer Stoffe sind Quarz, Diatomeenerde, Calciumsilikat, Zirkoniumsilikat, Zeolithe, Montmorrillonite, wie Bentonite, Metalloxidpulver, wie Aluminium-, Titan- oder Zinkoxide bzw. deren Mischoxide, Bariumsulfat, Calciumcarbonat, Gips, Glaspulver, pyrogen hergestellte und gefällte Kieselsäure und Silicium-Aluminium-Mischoxide. Die genannten anorganischen Stoffe können beispielsweise durch die Behandlung mit Organosilanen bzw. -siloxanen oder durch Verätherung von Hydroxylgruppen zu Alkoxygruppen hydrophobiert sein. Bevorzugt sind die pyrogen hergestellten und gefällten Kieselsäuren, da diese gut dispergierbar sind und die Beschichtung durchscheinend bleibt.

Als Wachse sind beispielsweise geeignet natürliche Wachse, wie pflanzliche Wachse, z.B. Candellila- und Carnaubawachs; tierische Wachse, z.B. Bienenwachs und Lanolin; Mineralwachse, z.B. Ceresin und Ozokerit; chemisch modifizierte natürliche, insbesondere fluorierte Wachse und synthetische Wachse, z.B. Polyethylenwachse und Siliconwachse.

Als Carbonsäuresalze eignen sich insbesondere die Salze von ein- oder mehrwertigen Carbonsäuren mit 8 bis 50 Kohlenstoffatomen pro Carboxylgruppe. Bevorzugt sind die Salze von fluorierten Carbonsäuren, insbesondere wenn diese einen Perfluoralkylrest mit mindestens 4 Kohlenstoffatomen aufweisen. Beispiele für bevorzugte einwertige fluorierte Carbonsäuresalze sind die Alkalimetallsalze von Arylcarbonsäuren, wie Benzoesäuren oder Naphthoesäuren mit einem oder zwei Perfluoralkylresten mit vorzugsweise 4 bis 18 Kohlenstoffatomen.

Als fluorierte organische polymere Verbindungen können im erfindungsgemäßen Verfahren beispielsweise alle derartigen Verbindungen eingesetzt werden, die auch bisher zum Wasser- und Ölabweisendmachen von organischen Stoffen, wie organischen Fasern, und anorganischen Stoffen verwendet werden konnten oder verwendet wurden. Beispiele für derartige Verbindungen sind aus mindestens zum Teil fluorhaltigen Monomeren hergestellte Polymere, wie Polytetrafluorethylen, Copolymere aus Tetrafluorethylen und Hexafluorpropylen, Polyvinylfluorid, Polyvinylidenfluorid, Polytrifluorchlorethylen, Copolymere aus Trifluorchlorethylen und anderen Monomeren, wie Vinylidenfluorid, Vinylchlorid, Vinylacetat, Methacrylsäuremethylester oder Styrol; und fluorierte Acrylharze, wie Homo- und Copolymere von Perfluoralkylgruppen enthaltenden Acrylsäure- und Methacrylsäureestern mit Acrylsäure und Methacrylsäure und deren vorstehend bei den filmbildenden Stoffen aufgeführten Derivaten.

Bevorzugte Beispiele für fluorierte Acrylharze sind Poly-1,1-dihydroperfluorbutylacrylat und die in der DE-C-25 26 287 beschriebenen Mischpolymerisate aus n-Butylacrylat, N-Methylolacrylamid und mindestens 35 Gew.-% 1,1,2,2,-Tetrahydroperfluor-C₁-bis C₁₆-alkylmethacrylat mit linearer Alkylkette.

Besonders bevorzugte fluorierte Acrylharze weisen mindestens so viele Carbonsäuresalzgruppen auf, daß die Harze wasserlöslich sind. Beispiele für solche wasserlöslichen Harze sind die Alkalimetallsalze der Copolymeren aus den vorstehend aufgeführten Acrylaten, Methacrylaten, Acrylsäure und Methacrylsäure, die vorzugsweise einen Fluorgehalt von mindestens 20 Gew.-% aufweisen.

Weitere Beispiele für fluorierte organische polymere Verbindungen sind nach der Polymerisation fluorierte organische synthetische Polymere, wie die bei den filmbildenden Stoffen aufgeführten Polymere, die in den Seitenketten oder in den Hauptketten fluoriert sind. Die nach der Polymerisation fluorierten Polymere weisen vorzugsweise einen Fluorgehalt von mindestens 10 Gew.-% auf. Besonders bevorzugt sind Polyurethanharze mit einem Fluorgehalt von 25-35 Gew.-%.

Die fluorierten organischen polymeren Verbindungen sind nicht nur wasserabweisend, sondern zusätzlich auch noch ölabweisend.

Wenn die wasserabweisenden Stoffe nicht wasserlöslich sind, werden vorzugsweise wasserlösliche filmbildende Stoffe eingesetzt, die die wasserabweisenden Stoffe dispergieren oder emulgieren können.

Vorzugsweise umfaßt die wässrige Zusammensetzung 0,1 bis 20 Gew.-%, insbesondere 1,0 bis 10 Gew.-% wasserabweisende Stoffe.

Als anionische Dispersionsmittel und Emulgatoren eignen sich im erfindungsgemäßen Verfahren beispielsweise:
1. Alkylsulfate, besonders solche mit einer Kettenlänge von 8 bis 18 C-Atomen, Alkyl- und Alkylethersulfate mit 8 bis 18 C-Atomen im hydrophoben Rest und 1 bis 40 Ethylenoxid(EO)- bzw. Propylenoxid(PO)einheiten.
2.Sulfonate, besonders Alkylsulfonate mit 8 bis 18 C-Atomen, Alkylarylsulfonate mit 8 bis 18 C-Atomen, Tauride, Ester und Halbester der Sulfobernsteinsäure mit einwertigen Alkoholen oder Alkylphenolen mit 4 bis 15 C-Atomen; ggf. können diese Alkohole oder Alkylphenole auch mit 1 bis 40 EO-Einheiten ethoxyliert sein.
3.Alkali- und Ammoniumsalze von Carbonsäuren mit 8 bis 20 C-atomen im Alkyl-, Aryl-, Alkaryl- oder Aralkylrest.
4.Phosphorsäureteilester und deren Alkali- und Ammoniumsalze, besonders Alkyl- und Alkarylphosphate mit 8 bis 20 C-Atomen im organischen Rest, Alkylether- bzw. Alkaryletherphosphate mit 8 bis 20 C-Atomen im Alkyl- bzw. Alkarylrest und 1 bis 40 EO-Einheiten.
   Als nichtionische Dispersionsmittel und Emulgatoren eignen sich im erfindungsgemäßen Verfahren beispielsweise:
5.Alkylpolyglycolether, vorzugsweise solche mit 8 bis 40 EO-Einheiten und Alkylresten von 8 bis 20 C-Atomen.
6.Alkylarylpolyglycolether, vorzugsweise solche mit 8 bis 40 EO-Einheiten und 8 bis 20 C-Atomen in den Alkyl- und Arylresten.
7.Ethylenoxid/Propylenoxid(EO/PO)-Blockcopolymere, vorzugsweise solche mit 8 bis 40 EO- bzw. PO-Einheiten.
8.Fettsäuren mit 6 bis 24 C-Atomen.
9.Naturstoffe und deren Derivate, wie Lecitin, Lanolin, Saponine, Cellulose; Cellulosealkylether und Carboxyalkylcellulosen, deren Alkylgruppen jeweils bis zu 4 Kohlenstoffatome besitzen.
10.Polare Gruppen enthaltende lineare Organo(poly)siloxane, insbesondere solche mit Alkoxygruppen mit bis zu 24 C-Atomen und/oder bis zu 40 EO- und/oder PO-Gruppen.
11.Der vorstehend beschriebene Polyvinylalkohol.
   Als kationische Dispersionsmittel und Emulgatoren eignen sich im erfindungsgemäßen Verfahren beispielsweise:
12.Salze von primären, sekundären und tertiären Fettaminen mit 8 bis 24 C-Atomen mit Essigsäure, Schwefelsäure, Salzsäure und Phosphorsäuren.
13.Quarternäre Alkylbenzolammoniumsalze, insbesondere solche, deren Alkylgruppe 6 bis 24 C-Atome besitzt, insbesondere die Halogenide, Sulfate, Phosphate und Acetate.
14.Alkylpyridinium-, Alkylimidazolinium- und Alkyloxazoliniumsalze, insbesondere solche, deren Alkylkette bis zu 18 C-Atome besitzt, speziell die Halogenide, Sulfate, Phosphate und Acetate.

Die wässrige Zusammensetzung kann für bestimmte Zwecke neben den vorstehenden Bestandteilen zusätzlich noch Tenside, Biozide, Duft- und/oder Farbstoffe enthalten. Die Biozide und Tenside werden vorzugsweise in Mengen von 0,05 bis 0,5 Gew.-% zugesetzt. Geeignete Biozide sind beispielsweise Fungizide, Bakterizide, Algicide und Mikrobicide.

Tenside umhüllen lipophile Verunreinigungen in den Beschichtungen und verhindern dadurch gemeinsam mit den vorstehend aufgeführten, gegebenenfalls anwesenden Dispersionsmitteln und Emulgatoren, daß die Verunreinigungen mit dem Substrat in Berührung kommen.

Als Tenside eignen sich besonders:
nichtionische Tenside, wie Oxalkylate, d.h. Reaktionsprodukte von Ethylenoxid und/oder Propylenoxid mit Alkoholen, Alkylphenolen oder Fettsäuren, Fettsäureethanolamide, Fettsäureglycerinester und Fettamine; Ester von Fettsäuren mit Polyhydroxyverbindungen; Blockcopolymere des Propylenoxids und Ethylenoxids; kationische Tenside, wie quarternäre Ammoniumverbindungen in denen das Stickstoffatom der Ammoniumgruppe an Alkyl-, Alkaryl-, Alkylenhydroxy-, Alkylenalkoxy- und Alkylenaminogruppen gebunden ist; an C- und/oder N-Atomen alkylierte Imidazoliniumsalze, und deren Anion Halogenid, Sulfat, Phosphat oder Acetat ist;
anionische Tenside, wie Carboxylate, speziell Alkali- oder Ammoniumsalze von Fettsäuren, von deren (Poly)oxyalkylenestern; Sulfonate, speziell Alkylbenzolsulfonate, Alkylnaphthalinsulfonate, Alkansulfonate, Olefinsulfonate, α-Sulfofettsäureester, Sulfobernsteinsäurealkylester, Alkoxy-, Acyloxy- und Acylaminoalkansulfonate; Sulfate, speziell primäre und sekundäre Alkylsulfate und Salze der Schwefelsäurehalbester von Alkyl- oder Alkylaryloligoglycolethern; Phosphonate und Phosphate, speziell Alkali und/oder Ammoniumsalze dieser Verbindungen, welche Alkyl- und/oder Alkarylgruppen aufweisen;
amphotere Tenside, wie Aminocarbonsäuren; Silicon-Tenside, insbesondere Polymethylsiloxane mit Ethylenoxy-, Propylenoxy-, Alkoxy- und Ammoniumgruppen; Fluortenside, insbesondere Fluoralkancarbonsäuren.

Die durch das erfindungsgemäße Verfahren aufgebrachte Beschichtung ist durch Wasser, insbesondere durch Abspritzen rückstandsfrei wieder entfernbar. Die Anwendung von Druck und die Verwendung von erwärmtem Wasser, vorzugsweise von 50 bis 95°C beschleunigt das Ablösen der Beschichtung. Trotz der Entfernbarkeit mit Wasser sind die nach dem erfindungsgemäßen Verfahren aufgebrachten Beschichtungen hinreichend witterungsbeständig, um über Monate durch die natürliche Beregnung nicht abgelöst zu werden.

Das erfindungsgemäße Verfahren ist auch zur Beschichtung poröser, wassersaugender Substrate geeignet, wenn diese vor der Beschichtung mit der wässrigen Zusammensetzung wasserabweisend behandelt werden. Die Beschichtung ist dann ebenfalls leicht und rückstandsfrei mit Wasser entfernbar.

Da die Beschichtung wasserdampfdurchlässig ist, kann bei Verwendung einer ebenfalls wasserdampfdurchlässigen Imprägnierung ein insgesamt wasserdampfdurchlässiges System erhalten werden. Beispiele für dafür geeignete bekannte Imprägnierungsmittel für poröse, wassersaugende Substratoberflächen sind die in der DE-A-25 58 184 (M. Roth; offengelegt am 7. 7. 1977 für Wacker-Chemie GmbH) aufgeführten Organosiliciumverbindungen, wie alkoxygruppenhaltige Organosilane, deren Teilhydrolysate, Umsetzungsprodukte aus Organochlorsilanen mit Ethylenglykol oder Diethylenglykol, Organopolysiloxane mit Alkoxy- und gegebenenfalls Hydroxylgruppen und Alkalikohlenwasserstoffsiliconate mit Alkoxy- und gegebenenfalls Hydroxylgruppen.

Als geeignete Imprägnierungsmittel sind auch wässrige Emulsionen von Alkyltrialkoxysilanen, stickstofffreien und basischen Stickstoff enthaltenden Polyorganosiloxanen mit Alkoxygruppen aus US-A-4 661 551 (H. Mayer et al.; ausgegeben am 28. 4. 1987 für Wacker-Chemie GmbH) bekannt. Wässrige Emulsionen von Alkyltrialkoxysilanen zum wasserabweisenden Imprägnieren von Baustoffen sind u.a. aus US-A-4 877 654 (M.E. Wilson; ausgegeben am 31. 10. 1989 für PCR Inc.) bekannt. Emulsionen von Alkoxygruppen enthaltenden Organopolysiloxanen zum Hydrophobieren von Baustoffen sind in US-A-4 704 416 (H. Eck und M. Roth; ausgegeben am 3. 5. 1988 für Wacker-Chemie GmbH) beschrieben.

Die Behandlung der wassersaugenden Substrate mit oleophoben Imprägnierungsmitteln beeinträchtigt deren Gas- und insbesondere Wasserdampfdurchlässigkeit und ist deshalb bei Baustoffen nicht bevorzugt.

Die nach dem erfindungsgemäßen Verfahren aufgebrachte Beschichtung ermöglicht die Entfernung von Verunreinigungen, wie Farbe, Teer, Dieselruß und Taubenschmutz von beispielsweise Metalloberflächen, Glasoberflächen, Kunststoffoberflächen, einschließlich elastomeren und thermoplastischen Kunststoffen und lackierten Flächen, imprägnierten Oberflächen von unbemaltem oder bemaltem Holz, Beton, Natur- oder Kunststein, Verputz und Keramik. Auch Plakate können leicht nach deren Durchfeuchtung abgezogen werden. Selbstklebende Aufkleber lassen sich leicht von den beschichteten Substratoberflächen abziehen.

In den nachfolgenden Beispielen sind, falls jeweils nicht anders angegeben,
a) alle Mengen- und Konzentrationsangaben auf das Gewicht bezogen;
b)alle Drücke 0,10 MPa (abs.);
c)alle Temperaturen 20°C.

### Beispiele

### Beispiel 1

Ein Gemisch aus 4,0 Teilen Polyvinylalkohol mit einer Verseifungszahl von 140, welches als 4,0 %ige Lösung bei 20°C eine Viskosität von 13 mPa.s aufweist, 5,0 Teilen einer 20 %igen Lösung eines 37 % Fluor enthaltenden Kaliumsalzes einer Arylmonocarbonsäure mit einem an der Arylgruppe gebundenen Perfluoralkylrest von durchschnittlich 7 Kohlenstoffatomen (Scotchgard FX 3535 von 3M Comp, USA) Wasser/Isopropanol/Butyl Cellosolve® (30/10/40) und 91,0 Gew.-Teilen Wasser, wurde auf die eine Hälfte einer PVC-Platte, einer Plexiglasplatte, auf Stahlblech, Alu-Blech und eloxiertes Alu-Blech mit dem Pinsel aufgetragen.

Nach 3 Tagen wurden die beiden Hälften der Platten mit Autolack sowie mit einem Autounterbodenschutz auf der Basis von Bitumen besprüht.

Nach weiteren 2 Tagen wurden die so behandelten Flächen mit Warmwasser unter leichtem Druck behandelt.

Während sich die beiden Verunreinigungen auf allen Substraten nach kurzer Einwirkungszeit des Wassers restlos ablösten, konnte bei den unbehandelten Flächen auch nach einer längeren Einwirkung des warmen Wassers kein Reinigungseffekt erzielt werden.

### Beispiel 2

Keramische Dachziegel, Kalksandsteine, Faserzement-Platten, Putzplatten sowie Natursteinplatten wurden zunächst mit einer hydrophob wirkenden Siliconlösung behandelt. Nach 24 Stunden wurde jeweils auf die Hälfte der so vorbehandelten Baustoffe die im Beispiel 1 genannte Mischung mit dem Pinsel aufgetragen. Nach weiteren 24 Stunden wurde auf beiden Hälften der Proben die im Beispiel 1 genannten Verunreinigungen sowie auch Graffiti mit Filzstift aufgebracht.

Nach einer Trocknungszeit von 4 Tagen wurden die Baustoffe mit Heißwasser unter schwachem Druck behandelt. Während im Falle der nicht behandelten Flächen alle drei Verunreinigungen sich nicht ablösten, waren die Verunreinigungen auf den erfindungsgemäß behandelten Flächen nach kurzer Einwirkungsdauer des heißen Wassers bei allen Baustoffen gut bis sehr gut zu entfernen.

### Beispiel 3

Um die Witterungsbeständigkeit der erfindungsgemäß aufgebrachten Beschichtungen zu testen, wurde auf die im Beispiel 2 beschriebenen Baustoffe, die vorher mit einer Siliconlösung hydrophobiert worden waren, mit einer 4,0 %igen Lösung des genannten Polyvinylalkohols (PVA), bzw. mit dem erfindungsgemäß verwendeten und in Beispiel 1 beschriebenen Gemisch behandelt.

Nach 2 Tagen wurde auf die beiden Flächen jeweils 0,1 ml Wasser bzw. Speiseöl aufgetropft.

Beurteilung der Wasser- bzw. Öltropfen:
Die mit der reinen PVA-Lösung behandelten Baustoffe konnten mit Wasser spontan sehr gut benetzt werden. Das Speiseöl wurde sehr schnell aufgesaugt.

Die erfindungsgemäß aufgebrachten Beschichtungen zeigten erst nach 2 Stunden eine beginnende Benetzung. Das Speiseöl war nach 24 Stunden noch nicht aufgesaugt.

Die starke hydrophobe und oleophobe Wirkung der erfindungsgemäß aufgebrachten Beschichtung verhindert ein schnelles Abwittern der Präventivmaßnahmen durch Niederschläge.

### Beispiel 4

97 Teile einer 4,0 %igen wäßrigen Lösung einer Carboxymethylcellulose, die in 2 %iger Lösung bei 20°C eine Viskosität von 25-32 mPa.s aufweist, sowie einer 2,0 %igen wäßrigen Lösung einer Carboxymethylcellulose, die in 2 %iger Lösung bei 20°C eine Viskosität von 200-330 mPa.s besitzt, wurde mit jeweils 3,0 Teilen einer Paste, die 40,0 Gew.-% fluoriertes Polyurethan mit einem Fluorgehalt von 31 Gew.-%, 40 % Wasser und 20 % Diethylenglykolmonobutylether enthält, vermischt.

Diese Mischungen wurden auf jeweils eine Hälfte, von vorher mit einer Siliconlösung wasserabweisend imprägnierten Ziegelplatten bzw. Kalksandsteine, mit dem Pinsel aufgetragen. Nach 4 Tagen wurde auf die ganzen Platten ein Autolack aufgesprüht. 2 Tage später wurden die Proben mit Heißwasser unter schwachem Druck behandelt. Während an den nur wasserabweisend imprägnierten Plattenhälften auch nach längerer Einwirkungszeit des Wassers keinerlei Reinigung festzustellen war, konnte an den mit den erfindungsgemäß behandelten Plattenhälften sehr schnell eine restlose Entfernung des Lackbelages erreicht werden.

### Beispiel 5

95,0 Teile einer 5,0 %igen wäßrigen Lösung einer Hydroxymethylcellulose, die als 2,0 %ige wäßrige Lösung bei 20°C eine Viskosität von 20 mPa.s aufweist, wurden mit 5,0 Teilen einer 30,0 %igen anionischen Dispersion des in Beispiel 1 verwendeten Kaliumsalzes in 60,0 % Wasser und 10,0 % Ethylenglykol vermischt.

Dieses Gemisch wurde auf Stahlblech sowie auf eine Glasplatte aufgesprüht. Nach 24 Stunden wurde Autolack, sowie ein auf Bitumenbasis aufgebauter Auto-Unterbodenschutz aufgesprüht.

2 Tage nach dem Aufsprühen des Lackes bzw. des Unterbodenschutzes wurden die so vorbereiteten Flächen unter schwachem Druck (Wasserleitungsdruck) mit Heißwasser beaufschlagt. Während die Verunreinigungen sich von den erfindungsgemäß vorbehandelten Proben sehr leicht restlos ablösten, waren die Proben ohne die erfindungsgemäße Vorbehandlung auch nach längerer Einwirkung des heißen Wassers nicht zu reinigen.

## Patentansprüche

1. Verfahren zur Beschichtung von nicht wassersaugenden Substratoberflächen, wobei die Substratoberflächen mit einer durch Behandlung mit Wasser wieder entfernbaren wässrigen Zusammensetzung beschichtet werden, die eine wäßrige Lösung, Dispersion und/oder Emulsion filmbildender Stoffe sowie wasserabweisender Stoffe umfaßt.

2. Verfahren nach Anspruch 1, wobei als filmbildende Stoffe organische Polymere eingesetzt werden.

3. Verfahren nach Anspruch 1 oder 2, wobei als filmbildende Stoffe Polysaccharide eingesetzt werden.

4. Verfahren nach einem der Ansprüche 1 bis 3, wobei als filmbildender Stoff Polyvinylalkohol eingesetzt wird.

5. Verfahren nach einem der Ansprüche 1 bis 4, wobei die wässrige Zusammensetzung 0,1 bis 20 Gew.-% filmbildende Stoffe umfaßt.

6. Verfahren nach einem der Ansprüche 1 bis 5, wobei als wasserabweisende Stoffe hydrophobierte anorganische Stoffe oder gegebenenfalls fluorierte Wachse, Paraffine, Carbonsäuresalze, organische oder siliziumorganische polymere Verbindungen eingesetzt werden.

7. Verfahren nach einem der Ansprüche 1 bis 6, wobei als wasserabweisende Stoffe fluorierte Carbonsäuresalze eingesetzt werden.

8. Verfahren nach einem der Ansprüche 1 bis 7, wobei die Zusammensetzung zusätzlich noch Tenside, Biozide, Duft- und/oder Farbstoffe umfaßt.

9. Verfahren nach einem der Ansprüche 1 bis 8, wobei die Zusammensetzung 0,1 bis 20 Gew.-% wasserabweisende Stoffe umfaßt.

10. Verfahren nach einem der Ansprüche 1 bis 9, wobei poröse, wassersaugende Substrate vor der Beschichtung mit der wässrigen Zusammensetzung wasserabweisend behandelt werden.
